# EUROPEAN PATENT APPLICATION

(11) **EP 4 603 046 A2**
(43) Date of publication of application: **20.08.2025**
(21) Application number: 25186934.3
(22) Date of filing: 24.05.2022
(51) Int. Cl.: A61B 18/00

(54) **SYSTEMS AND METHODS FOR ELECTROPORATION DEVICES INCLUDING BASKET AND BALLOON CONFIGURATIONS**

(30) Priority: 25.05.2021 US 202163192723 P
(62) Divisional of application: 22730013.4
(71) Applicant: St. Jude Medical, Cardiology Division, Inc., St. Paul, MN 55117 (US)
(72) Inventor: ZHANG, Sean, St. Paul, 55117 (US); MARASS, Timothy S., St. Paul, 55117 (US); TEGG, Troy, St. Paul, 55117 (US); DALY, Jake, St. Paul, 55117 (US); SUTERMEISTER, Derek, St. Paul, 55117 (US); NEUDECKER, Wayne, St. Paul, 55117 (US); ARIAS, Salo, St. Paul, 55117 (US)
(74) Representative: Mathys & Squire

(57) **Abstract**

Systems and methods for electroporation catheters are disclosed herein. An electroporation catheter includes a shaft, and a plurality of splines forming a basket around a distal portion of the shaft, each spline extending between a proximal end that is coupled to the shaft and a distal end that is coupled to the shaft, wherein each spline of the plurality of splines comprises at least one energizable electrode. The electroporation catheter further includes a balloon positioned within the basket formed by the plurality of splines, the balloon selectively inflatable to facilitate securing a position of the plurality of splines.

## Description

### CROSS REFERENCE TO RELATED APPLICATIONS

This application claims priority to provisional application serial No. 63/192,723, filed May 25, 2021, which is incorporated herein by reference in its entirety.

### FIELD OF THE DISCLOSURE

The present disclosure relates generally to tissue ablation systems. In particular, the present disclosure relates to electroporation catheters including catheter assemblies with baskets and/or balloons.

### BACKGROUND

It is generally known that ablation therapy may be used to treat various conditions afflicting the human anatomy. For example, ablation therapy may be used in the treatment of atrial arrhythmias. When tissue is ablated, or at least subjected to ablative energy generated by an ablation generator and delivered by an ablation catheter, lesions form in the tissue. Electrodes mounted on or in ablation catheters are used to create tissue apoptosis in cardiac tissue to correct conditions such as atrial arrhythmia (including, but not limited to, ectopic atrial tachycardia, atrial fibrillation, and atrial flutter).

Arrhythmia (i.e., irregular heart rhythm) can create a variety of dangerous conditions including loss of synchronous atrioventricular contractions and stasis of blood flow which can lead to a variety of ailments and even death. It is believed that the primary cause of atrial arrhythmia is stray electrical signals within the left or right atrium of the heart. The ablation catheter imparts ablative energy (e.g., radiofrequency energy, cryoablation, lasers, chemicals, high-intensity focused ultrasound, etc.) to cardiac tissue to create a lesion in the cardiac tissue. This lesion disrupts undesirable electrical pathways and thereby limits or prevents stray electrical signals that lead to arrhythmias.

Electroporation is a non-thermal ablation technique that involves applying strong electric-fields that induce pore formation in the cellular membrane. The electric field may be induced by applying a relatively short duration pulse which may last, for instance, from a nanosecond to several milliseconds. Such a pulse may be repeated to form a pulse train. When such an electric field is applied to tissue in an in vivo setting, the cells in the tissue are subjected to trans-membrane potential, which opens the pores on the cell wall. Electroporation may be reversible (i.e., the temporally-opened pores will reseal) or irreversible (i.e., the pores will remain open). For example, in the field of gene therapy, reversible electroporation (i.e., temporarily open pores) is used to transfect high molecular weight therapeutic vectors into the cells. In other therapeutic applications, a suitably configured pulse train alone may be used to cause cell destruction, for instance by causing irreversible electroporation.

For catheters used to deliver bipolar energy using irreversible electroporation (IRE) or pulsed field ablation (PFA) it is important to ensure that catheter electrodes are close to or contacting a vessel wall. Generally, the closer the electrodes to the vessel wall, the larger the lesion size. Accordingly, catheter configurations that place electrodes near or in contact with the vessel wall are desirable.

### BRIEF SUMMARY OF THE DISCLOSURE

In one aspect, an electroporation catheter is provided. The electroporation catheter includes a shaft, and a plurality of splines forming a basket around a distal portion of the shaft, each spline extending between a proximal end that is coupled to the shaft and a distal end that is coupled to the shaft, wherein each spline of the plurality of splines comprises at least one energizable electrode. The electroporation catheter further includes a balloon positioned within the basket formed by the plurality of splines, the balloon selectively inflatable to facilitate securing a position of the plurality of splines.

In another aspect, an electroporation system is provided. The system includes a generator and a catheter coupled to the generator. The catheter includes a handle, a shaft extending distally from the handle, and a plurality of splines forming a basket around a distal portion of the shaft, each spline extending between a proximal end that is coupled to the shaft and a distal end that is coupled to the shaft, wherein each spline of the plurality of splines comprises at least one energizable electrode. The catheter further includes a balloon positioned within the basket formed by the plurality of splines, the balloon selectively inflatable to facilitate securing a position of the plurality of splines.

In yet another aspect, a method of assembling an electroporation catheter is provided. The method includes providing a shaft, coupling a plurality of splines to the shaft to form a basket around a distal portion of the shaft, each spline extending between a proximal end that is coupled to the shaft and a distal end that is coupled to the shaft, wherein each spline of the plurality of splines includes at least one energizable electrode, and positioning a balloon within the basket formed by the plurality of splines, the balloon selectively inflatable to facilitate securing a position of the plurality of splines.

The foregoing and other aspects, features, details, utilities and advantages of the present disclosure will be apparent from reading the following description and claims, and from reviewing the accompanying drawings.

### BRIEF DESCRIPTION OF THE DRAWINGS

Figure 1 is a schematic and block diagram view of a system for electroporation therapy.
Figure 2 is a view of one embodiment of a handle that may be used with the system shown in Figure 1.
Figure 3 is a side schematic view of one embodiment of a catheter assembly that may be used with the system shown in Figure 1.
Figure 4 is a side schematic view of an alternative embodiment of a catheter assembly that may be used with the system shown in Figure 1.
Figure 5 is a side schematic view of an alternative embodiment of a catheter assembly that may be used with the system shown in Figure 1.
Figure 6 is a side schematic view of an alternative embodiment of a catheter assembly that may be used with the system shown in Figure 1.
Figure 7 is a side schematic view of an inner lumen that may be used with the catheter assembly shown in Figure 6.
Figure 8 is a perspective schematic view showing splines deployed from the inner lumen shown in Figure 7.
Figure 9A is a perspective view of an alternative embodiment of a catheter assembly.
Figure 9B is a side schematic view of the catheter assembly shown in Figure 9A.
Figure 10A is a perspective view of an alternative embodiment of a catheter assembly.
Figure 10B is a side schematic view of the catheter assembly shown in Figure 10A.
Figure 10C is a schematic diagram showing the catheter assembly of Figure 10A in a first configuration.
Figure 10D is a schematic diagram showing the catheter assembly of Figure 10A in a first configuration.
Figure 10E is a side cross-sectional view of the catheter assembly shown in Figure 10A.
Figures 10F-10J are schematic side cross-sectional views of the catheter assembly shown in Figure 10A illustrating the transitioning of the catheter assembly between different states.
Figure 10K is a side cross-sectional view of a portion of a handle that may be used with the catheter assembly shown in Figure 10A.
Figure 10L is a perspective view of a compression valve component, pin, and ring that may be used with the handle shown in Figure 10K.
Figure 10M is a perspective exploded view of the compression valve component, pin, and ring shown in Figure 10L.
Figure 11 is a perspective view of an alternative embodiment of a catheter assembly.
Figure 12 is a perspective view of an alternative embodiment of a catheter assembly.
Figure 13 is a perspective view of an alternative embodiment of a catheter assembly.
Figure 14 is a perspective view of an alternative embodiment of a catheter assembly.
Figure 15 is a perspective view of an alternative embodiment of a catheter assembly.
Figure 16 is a perspective view of an alternative embodiment of a catheter assembly.
Figure 17 is a perspective view of an alternative embodiment of a catheter assembly.
Figure 18 is a perspective view of an alternative embodiment of a catheter assembly.
Figure 19 is a perspective view of an alternative embodiment of a catheter assembly.

### DETAILED DESCRIPTION OF THE DISCLOSURE

Systems and methods for electroporation catheters are described herein. An example electroporation catheter includes a shaft, and a plurality of splines forming a basket around a distal portion of the shaft, each spline extending between a proximal end that is coupled to the shaft and a distal end that is coupled to the shaft, wherein each spline of the plurality of splines comprises at least one energizable electrode. The electroporation catheter further includes a balloon positioned within the basket formed by the plurality of splines, the balloon selectively inflatable to facilitate securing a position of the plurality of splines.

Although exemplary embodiments of the present disclosure are described with respect to pulmonary vein isolation (PVI), it is contemplated that the described features and methods of the present disclosure as described herein may be incorporated into any number of systems and any number of applications as would be appreciated by one of ordinary skill in the art based on the disclosure herein.

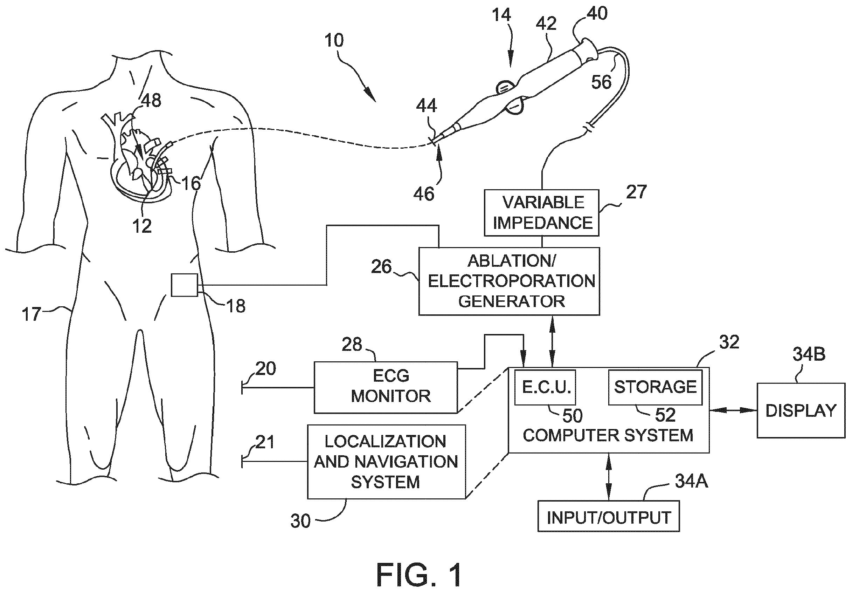

Figure 1A is a block diagram view of a system 10 for electroporation therapy. In general, system 10 includes a catheter electrode assembly 12 disposed at a distal end 48 of a catheter 14. As used herein, "proximal" refers to a direction toward the end of the catheter near the clinician and "distal" refers to a direction away from the clinician and (generally) inside the body of a patient. The electrode assembly includes one or more individual, electrically-isolated electrode elements. Each electrode element, also referred to herein as a catheter electrode, is individually wired such that it can be selectively paired or combined with any other electrode element to act as a bipolar or a multi-polar electrode.

System 10 may be used for irreversible electroporation (IRE) to destroy tissue. In particular, system 10 may be used for electroporation-induced primary apoptosis therapy, which refers to the effects of delivering electrical current in such a manner as to directly cause an irreversible loss of plasma membrane (cell wall) integrity leading to its breakdown and cell apoptosis. This mechanism of cell death may be viewed as an "outside-in" process, meaning that the disruption of the outside wall of the cell causes detrimental effects to the inside of the cell. Typically, for classical plasma membrane electroporation, electric current is delivered as a pulsed electric field in the form of short-duration pulses (e.g., having a 0.1 to 20 millisecond (ms) duration) between closely spaced electrodes capable of delivering an electric field strength of about 0.1 to 1.0 kilovolts/centimeter (kV/cm). System 10 may be used, for example, with a basket and/or balloon catheter assembly for high output (e.g., high voltage and/or high current) electroporation procedures. In some particular embodiments, system 10 is configured to deliver an electroporation pulse signal having a relatively high voltage and low pulse duration.

In one embodiment, all electrodes of the catheter deliver an electric current simultaneously. Alternatively, in other embodiments, stimulation is delivered between pairs of electrodes on the catheter. Delivering electric current simultaneously using a plurality of electrodes may facilitate creating a sufficiently deep lesion for electroporation. To facilitate activating electrodes simultaneously, the electrodes may be switchable between being connected to a 3D mapping system and being connected to EP amplifiers.

Irreversible electroporation using the catheters described herein may enable pulmonary vein isolation in as few as one shock per vein, which may produce much shorter procedure times compared to sequentially positioning a radiofrequency (RF) ablation tip around a vein.

It should be understood that while the energization strategies are described as involving DC pulses, embodiments may use variations and remain within the spirit and scope of the disclosure. For example, exponentially-decaying pulses, exponentiallyincreasing pulses, and combinations may be used. Further, in some embodiments, AC pulses may be used.

Further, it should be understood that the mechanism of cell destruction in electroporation is not primarily due to heating effects, but rather to cell membrane disruption through application of a high-voltage electric field. Thus, electroporation may avoid some possible thermal effects that may occur when using radio frequency (RF) energy. This "cold therapy" thus has desirable characteristics.

With this background, and now referring again to Figure 1, system 10 includes a catheter electrode assembly 12 including at least one catheter electrode. Electrode assembly 12 is incorporated as part of a medical device such as a catheter 14 for electroporation therapy of tissue 16 in a body 17 of a patient. In the illustrative embodiment, tissue 16 includes heart or cardiac tissue. It should be understood, however, that embodiments may be used to conduct electroporation therapy with respect to a variety of other body tissues.

Figure 1 further shows a plurality of return electrodes designated 18, 20, and 21, which are diagrammatic of the body connections that may be used by the various sub-systems included in overall system 10, such as an electroporation generator 26, an electrophysiology (EP) monitor such as an ECG monitor 28, and a localization and navigation system 30 for visualization, mapping, and navigation of internal body structures. In the illustrated embodiment, return electrodes 18, 20, and 21 are patch electrodes. It should be understood that the illustration of a single patch electrode is diagrammatic only (for clarity) and that such sub-systems to which these patch electrodes are connected may, and typically will, include more than one patch (body surface) electrode, and may include split patch electrodes (as described herein). In other embodiments, return electrodes 18, 20, and 21 may be any other type of electrode suitable for use as a return electrode including, for example, one or more catheter electrodes. Return electrodes that are catheter electrodes may be part of electrode assembly 12 or part of a separate catheter or device (not shown). System 10 may further include a main computer system 32 (including an electronic control unit 50 and data storage-memory 52), which may be integrated with localization and navigation system 30 in certain embodiments. System 32 may further include conventional interface components, such as various user input/output mechanisms 34A and a display 34B, among other components.

Electroporation generator 26 is configured to energize the electrode element(s) in accordance with an electroporation energization strategy, which may be predetermined or may be user-selectable. For electroporation-induced primary apoptosis therapy, generator 26 may be configured to produce an electric current that is delivered via electrode assembly 12 as a pulsed electric field in the form of short-duration DC pulses (e.g., a nanosecond to several milliseconds duration, a 0.1 to 20 ms duration, or any duration suitable for electroporation) between closely spaced electrodes capable of delivering an electric field strength (i.e., at the tissue site) of about 0.1 to 1.0 kV/cm. The amplitude and pulse duration needed for irreversible electroporation are inversely related. As pulse durations are decreased, the amplitude must be increased to achieve electroporation.

Electroporation generator 26, sometimes also referred to herein as a DC energy source, is a monophasic electroporation generator 26 configured to generate a series of DC energy pulses that all produce current in the same direction. In other embodiments, electroporation generator is biphasic or polyphasic electroporation generator configured to produce DC energy pulses that do not all produce current in the same direction. In some embodiments, electroporation generator 26 is configured to output energy in DC pulses at selectable energy levels, such as fifty joules, one hundred joules, two hundred joules, and the like. Other embodiments may have more or fewer energy settings and the values of the available setting may be the same or different. For successful electroporation, some embodiments utilize the two hundred joule output level. For example, electroporation generator 26 may output a DC pulse having a peak magnitude from about 300 Volts (V) to about 3,200 V at the two hundred joule output level. In some embodiments, the peak magnitude may be even larger (e.g., on the order of 10,000 V). Other embodiments may output any other suitable positive or negative voltage. For example, in some embodiments, the systems and methods described herein may include pulses with amplitudes from about 500 V to about 4,000 V, with pulse widths from about 200 nanoseconds to about 20 microseconds.

In some embodiments, a variable impedance 27 allows the impedance of system 10 to be varied to limit arcing. Moreover, variable impedance 27 may be used to change one or more characteristics, such as amplitude, duration, pulse shape, and the like, of an output of electroporation generator 26. Although illustrated as a separate component, variable impedance 27 may be incorporated in catheter 14 or generator 26.

With continued reference to Figure 1, as noted above, catheter 14 may include functionality for electroporation and in certain embodiments also other types of ablation (e.g., RF ablation). It should be understood, however, that in those embodiments, variations are possible as to the type of ablation energy provided (e.g., cryoablation, ultrasound, etc.).

In the illustrative embodiment, catheter 14 includes a cable connector or interface 40, a handle 42, and a shaft 44 having a proximal end 46 and a distal 48 end. Catheter 14 may also include other conventional components not illustrated herein such as a temperature sensor, additional electrodes, and corresponding conductors or leads. Connector 40 provides mechanical and electrical connection(s) for cable 56 extending from generator 26. Connector 40 may include conventional components known in the art and as shown is disposed at the proximal end of catheter 14.

Handle 42 provides a location for the clinician to hold catheter 14 and may further provide means for steering or the guiding shaft 44 within body 17. For example, handle 42 may include means to change the length of a guidewire extending through catheter 14 to distal end 48 of shaft 44 or means to steer shaft 44. Moreover, in some embodiments, handle 42 may be configured to vary the shape, size, and/or orientation of a portion of the catheter, and it will be understood that the construction of handle 42 may vary. In an alternate embodiment, catheter 14 may be robotically driven or controlled. Accordingly, rather than a clinician manipulating a handle to advance/retract and/or steer or guide catheter 14 (and shaft 44 thereof in particular), a robot is used to manipulate catheter 14. Shaft 44 is an elongated, tubular, flexible member configured for movement within body 17. Shaft 44 is configured to support electrode assembly 12 as well as contain associated conductors, and possibly additional electronics used for signal processing or conditioning. Shaft 44 may also permit transport, delivery and/or removal of fluids (including irrigation fluids and bodily fluids), medicines, and/or surgical tools or instruments. Shaft 44 may be made from conventional materials such as polyurethane and defines one or more lumens configured to house and/or transport electrical conductors, fluids or surgical tools, as described herein. Shaft 44 may be introduced into a blood vessel or other structure within body 17 through a conventional introducer. Shaft 44 may then be advanced/retracted and/or steered or guided through body 17 to a desired location such as the site of tissue 16, including through the use of guidewires or other means known in the art.

In some embodiments, catheter 14 includes a basket catheter assembly having catheter electrodes (not shown in Figure 1) distributed at the distal end of shaft 44 in a basket structure. Further, as described herein, an inflatable balloon may be contained within the basket structure.

Localization and navigation system 30 may be provided for visualization, mapping and navigation of internal body structures. Localization and navigation system 30 may include conventional apparatus known generally in the art (e.g., an EnSite Precision^{™} System, commercially available from Abbott Laboratories. and as generally shown with reference to commonly assigned U.S. Pat. No. 7,263,397 titled "Method and Apparatus for Catheter Navigation and Location and Mapping in the Heart," the entire disclosure of which is incorporated herein by reference). It should be understood, however, that this system is an example only, and is not limiting in nature. Other technologies for locating/navigating a catheter in space (and for visualization) are known, including for example, the CARTO navigation and location system of Biosense Webster, Inc., the Rhythmia^{®} system of Boston Scientific Scimed, Inc., the KODEX^{®} system of Koninklijke Philips N.V., the AURORA^{®} system of Northern Digital Inc., commonly available fluoroscopy systems, or a magnetic location system such as the gMPS system from Mediguide Ltd. In this regard, some of the localization, navigation and/or visualization system would involve a sensor be provided for producing signals indicative of catheter location information, and may include, for example one or more electrodes in the case of an impedance-based localization system, or alternatively, one or more coils (i.e., wire windings) configured to detect one or more characteristics of a magnetic field, for example in the case of a magnetic-field based localization system. As yet another example, system 10 may utilize a combination electric field-based and magnetic field-based system as generally shown with reference to U.S. Pat. No. 7,536,218 entitled "Hybrid Magnetic-Based and Impedance Based Position Sensing," the disclosure of which is incorporated herein by reference in its entirety.

Pulsed field ablation (PFA) has been shown to be an effective form of ablation for treatment of cardiac arrhythmias, particularly for instantaneous pulmonary vein isolation (PVI). PFA includes delivering high voltage pulses from electrodes disposed on a catheter (e.g., including the basket and/or balloon catheters described herein). In PFA, for example, voltage amplitudes may range from about 300 V to at least 3,200 V (or even as large as on the order as 10,000 V), and pulse widths may from hundreds of nanoseconds to tens of milliseconds.

These electric fields may be applied between adjacent electrodes (in a bipolar approach) or between a one or more electrodes and a return patch (in a monopolar approach). There are advantages and disadvantages to each of these approaches (e.g., when using the basket and/or balloon catheters described herein).

For example, regarding lesion contiguity, the monopolar approach has the potential to leave gaps in lesion coverage (referred to as dead zones) between electrodes where the field strength is low or zero, whereas the field strength in the bipolar approach generally prevents dead zones between electrodes.

For lesion size and proximity, the monopolar approach has a wider range of effect, and can potentially create deeper lesions with the same applied voltage. Further, the monopolar approach may be able to create lesions from a distance (e.g., generally proximate, but not necessarily contacting tissue). The bipolar approach may create smaller lesions, requiring closer proximity or contact with tissue to create transmural lesions. However, the monopolar approach may create larger lesions than are necessary, while the lesions generated using the bipolar approach may be more localized.

Due to a wider range of effect, the monopolar approach may cause unwanted skeletal muscle and/or nerve activation. In contrast, the bipolar approach has a constrained range of effect proportional to electrode spacing on the lead, and is less likely to depolarize cardiac myocytes or nerve fibers.

For the monopolar approach, only a single potential is applied in catheter wires and electrodes. Further, because all the electrodes are at the same polarity, the configuration is not susceptible to arcing (e.g., when using the basket and/or balloon catheters described herein). In contrast, for the bipolar approach, the internal architecture of the catheter must be constructed to prevent arcing, as different electrodes are at different potentials.

To monitor operation of system 10, one or more impedances between catheter electrodes and/or return electrodes 18, 20, and 21 may be measured. For example, for system 10, impedances may be measured as described in U.S. Patent Application Publication No. 2019/0117113, filed on October 23, 2018, U.S. Patent Application Publication No. 2019/0183378, filed on December 19, 2018, and U.S. Patent Application No. 63/027,660, filed on May 20, 2020, all of which are incorporated by reference herein in their entirety.

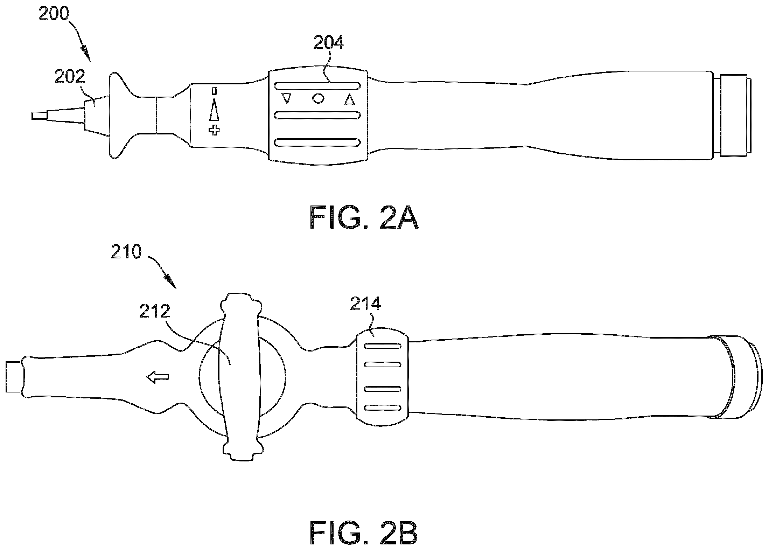

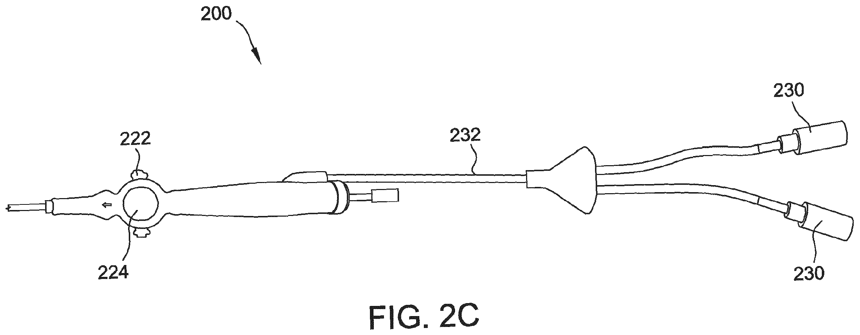

Figure 2 is a view of one embodiment of a handle 200 that may be used with system 10 and catheter 14. Handle 200 includes a first actuator 222 and a second actuator 224. First actuator 222 may be, for example, rotatable about a rotational axis that is substantially perpendicular to a longitudinal axis of handle 200 to selectively deflect at least a portion of catheter 14. Second actuator 224 may be, for example, rotatable about the same axis as first actuator 222 to selectively lock in a deflected orientation of catheter 14. That is, first actuator 222 may be manipulated to deflect at least a portion of catheter 14 to a desired orientation, and then second actuator 224 may be manipulated to lock catheter 14 in that orientation. Accordingly, first and second actuators 222 and 224 may be connected to one or more activation wires extending through catheter 14.

Further, as shown in Figure 2, a plurality of connectors 230 are coupled to handle 200 by a cable 232. Connectors 230 may be used to connect catheter 14 to a generator, such as generator 26 (shown in Figure 1). Further, connectors 230 may provide an interface between a localization and navigation system, such as localization and navigation system 30 (shown in Figure 1), and one or more magnetic sensors included on catheter 14, as described in more detail below.

Those of skill in the art will appreciate that handle 200 is merely an example, and that any suitable handles and/or arrangement of actuators may be used to implement the systems and methods described herein.

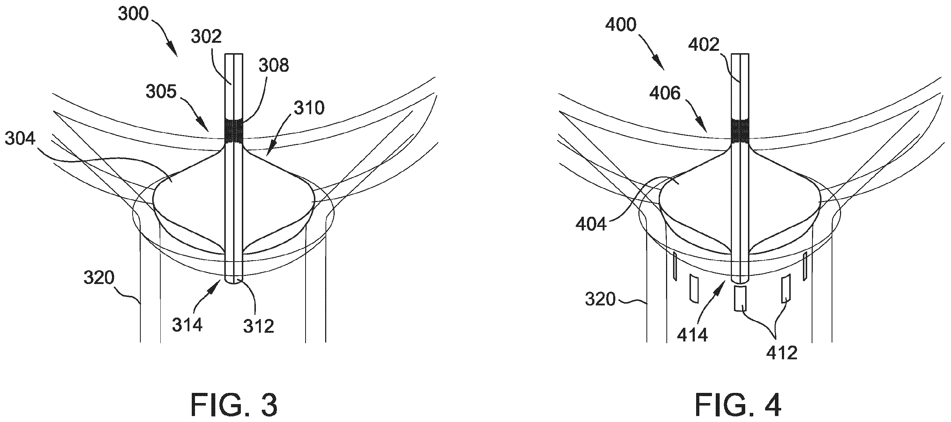

Figure 3 is a side schematic view of one embodiment of a catheter assembly 300 that may be used with system 10 (shown in Figure 1). Catheter assembly 300 includes a shaft 302 and a balloon 304 coupled to a distal end 306 of shaft 302. In this embodiment, catheter 300 includes a first electrode 308 at a proximal end 310 of balloon 304, and a second electrode 312 at a distal end 314 of balloon 304. Catheter assembly 300 is shown positioned within a pulmonary vein 320.

Figure 4 is a side schematic view of an alternative embodiment of a catheter assembly 400 that may be used with system 10 (shown in Figure 1). Catheter assembly 400 includes a shaft 400 and a balloon 404 coupled to a distal end 406 of shaft 402. In this embodiment, catheter 400 includes a plurality of electrodes 412 proximate a distal end 414 of balloon 404. Unlike second electrode 312 (shown in in Figure 3), electrodes 412 expand outward from balloon 404 to be closer to the wall of pulmonary vein 320. In this embodiment, catheter assembly 400 includes eight electrodes 412 (for clarity, only five are shown). Alternatively, catheter assembly 400 may include any suitable number of electrodes 412.

In this embodiment, each electrode 412 is coupled to a corresponding spline (not shown). During delivery, the splines and electrodes 412 fit into an inner lumen of catheter assembly 400. Upon deployment of catheter assembly 400, splines expand outward (e.g., similar to an umbrella) such that electrodes 412 are proximate, or even contacting, the wall of pulmonary vein 320. Using catheter assembly 400, sufficient lesions may be generated at an applied voltage of 1,400 V to 2,500 V, even for a 25 mm diameter pulmonary vein.

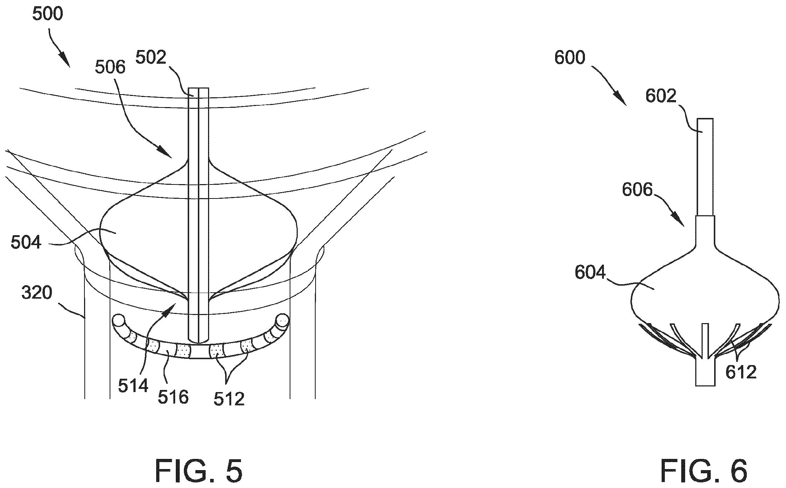

Figure 5 is a side schematic view of an alternative embodiment of a catheter assembly 500 that may be used with system 10 (shown in Figure 1). Catheter assembly 500 includes a shaft 502 and a balloon 504 coupled to a distal end 506 of shaft 502. In this embodiment, catheter assembly 500 includes a plurality of electrodes 512 proximate a distal end 514 of balloon 504. Unlike second electrode 312 (shown in in Figure 3), electrodes 512 are arranged on a loop 516 to be closer to the wall of pulmonary vein 320. During delivery, loop 516 and electrodes 512 fit into an inner lumen of catheter assembly 500. Upon deployment of catheter assembly 400, loop 516 deploys such that electrodes 512 are proximate, or even contacting, the wall of pulmonary vein 320.

In this embodiment, catheter assembly 500 includes fourteen electrodes 512 (for clarity, only half of loop 514 and eight electrodes 512 are shown). Alternatively, catheter assembly 500 may include any suitable number of electrodes 512. Using catheter assembly 500, sufficient lesions may be generated at an applied voltage of 2,000 V or 2,500 V, even for a 25 mm diameter pulmonary vein.

Figure 6 is a side schematic view of an alternative embodiment of a catheter assembly 600 that may be used with system 10 (shown in Figure 1). Catheter assembly 600 includes a shaft 602 and a balloon 604 coupled to a distal end 606 of shaft 602. In this embodiment, catheter assembly 600 includes a plurality of pre-shaped splines 612 that function as electrodes. During delivery, splines 612 may fit into an inner lumen of catheter assembly 600, or may surround balloon 604. In implementations where splines 612 fit into the inner lumen, splines 612 may expand radially outward when deployed such that splines 612 are proximate, or even contacting, the wall of pulmonary vein 320.

In the embodiment shown, catheter assembly 600 includes twelve splines 612 (although only seven splines 612 are shown). Alternatively, catheter assembly 600 may include any suitable number of splines 612. For example, catheter assembly 600 may include ten to sixteen splines 612 in some embodiments.

Splines 612 may be fabricated from Nitinol, stainless steel, and/or other superalloys. Further, the entirety of each spline 612 may serve as an electrode, or portions of each spline 612 may be covered with an insulating material (e.g., a polyethylene terephthalate (PET) heat shrink material or polyether block amide (PEBA) material) such that only non-insulation portions of each spline 612 serve as electrodes. Further, in some implementations, multiple independently energizable electrodes are attached to each spline 612 Using catheter assembly 600, sufficient lesions may be generated at an applied voltage of 2,000 V or 2,500 V, even for a 25 mm diameter pulmonary vein.

Splines 612 may be formed, for example, by laser cutting a tubing (e.g., made of Nitinol, stainless steel, and/or other superalloys) into multiple strips, and heat treating the strips to form a shape that conforms with balloon 604.

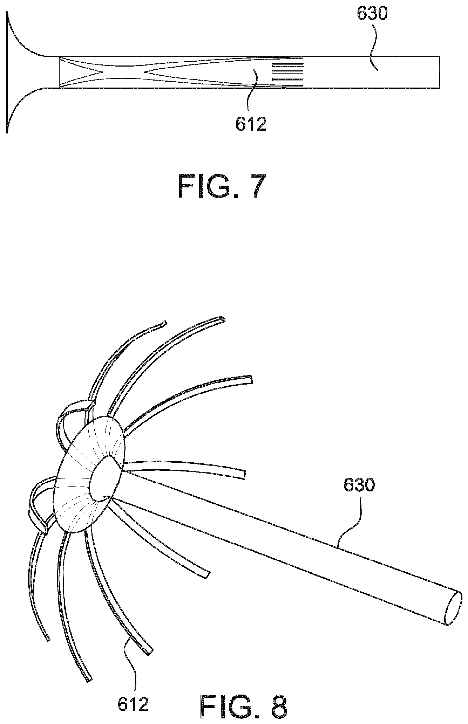

Figure 7 is a side schematic view of an inner lumen 630 of catheter assembly 600, with splines 612 stored inside inner lumen 630 (e.g., during delivery of catheter assembly 600 and prior to deployment of splines 612). Figure 8 is a perspective schematic view showing splines 612 deployed from inner lumen 630.

Splines 612 may all be electrically connected to one another as a single electrode, or may each be individual electrodes. When splines 612 are individual electrodes, each spline 612 may be selectively energizable to form different energization sequences and/or patterns. In generally, each spline 612 has the same polarity, to avoid arcing issues.

Splines 612 may all have the same length, or at least some of splines 612 may have different lengths. Further, splines 612 may include insulation covering at least a portion of each spline 612. The insulation on each spline 612 may have the same length, or at least some splines 612 may have insulation with different lengths. In addition, in some embodiments, catheter assembly 600 includes a distal electrode (not shown) positioned distal of splines 612. The distal electrode may be used to perform point ablation (e.g., by creating a bipole between the distal electrode and one of splines 612), and/or may be used for visualization/mapping purposes (e.g., using the distal electrode in combination with an electrode on shaft 602).

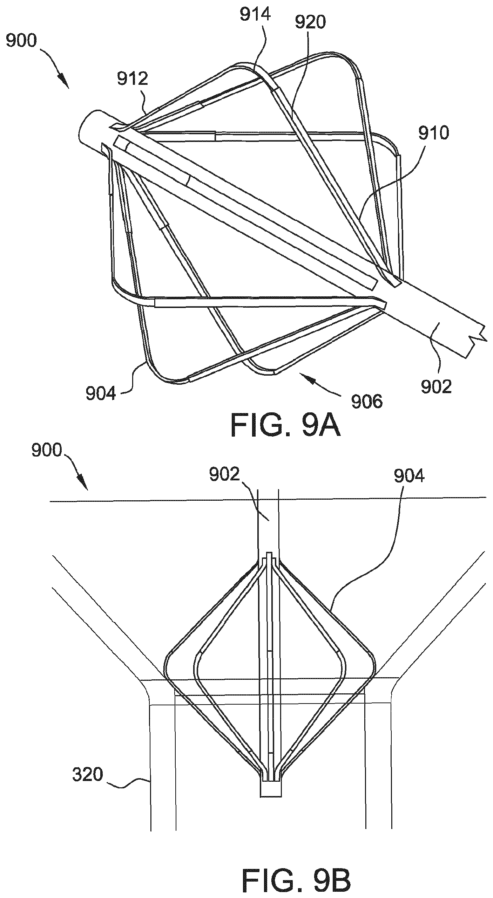

Figure 9A is a perspective view of an alternative embodiment of a catheter assembly 900, and Figure 9B is a side schematic view of catheter assembly 900. Catheter assembly 900 includes a shaft 902 and a plurality of splines 904 surrounding a distal portion 906 of shaft 902. Each spline 904 includes a proximal end 910 coupled to shaft 902 and a distal end 912 coupled to shaft 902. From proximal end 910, spline 904 extends radially outward to an inflection point 914, and then extends radially inward to distal end 912. Figure 9B shows catheter assembly 900 positioned within pulmonary vein 320.

A body of each spline 904 is made of an elastic material (e.g., Nitinol), and functions as a relatively large electrode. In this embodiment, alternating splines 904 alternate polarities. That is, each positive spline 904 is positioned between two negative splines 904 and vice-versa. Alternatively, any suitable polarization scheme may be used.

To control the ablation zone of each spline 904, portions of each spline 904 may be covered with insulating material 920 (e.g., heat-shrink (e.g., PET) or polymer tubing or spray or dip coat with polyimide or PEBA), and the exposed portions of splines 904 function as electrodes. In the embodiment shown in Figures 9A and 9B, inflection point 914 and portions of spline 904 between inflection point 914 and distal end 912 are generally exposed, while portions of spline 904 between inflection point 914 and proximal end 910 are generally insulated. This results in the portions of spline 904 that contact pulmonary vein 320 being exposed (see Figure 9B). Alternatively, any suitable insulation configuration may be used.

During delivery, splines 904 may be collapsed and be oriented substantially parallel to shaft 902 (i.e., with inflection point 914 proximate shaft 902). Subsequently, to perform ablation, splines 904 are deployed with inflection points 914 extending radially outward.

Notably, as compared to catheter assemblies 300, 400, 500, and 600 (shown in Figures 3, 4, 5, and 6), catheter assembly 900 facilitates ablating a more proximal, and wider portion of pulmonary vein 320.

Splines 904 may all have the same length, or at least some of splines 904 may have different lengths. Further, insulating material 920 on each spline 904 may have the same length, or at least some splines 904 may have insulating material 920 with different lengths. In addition, in some embodiments, catheter assembly 900 includes a distal electrode (not shown) positioned distal of splines 904. The distal electrode may be used to perform point ablation (e.g., by creating a bipole between the distal electrode and one of splines 904), and/or may be used for visualization/mapping purposes (e.g., using the distal electrode in combination with an electrode on shaft 902).

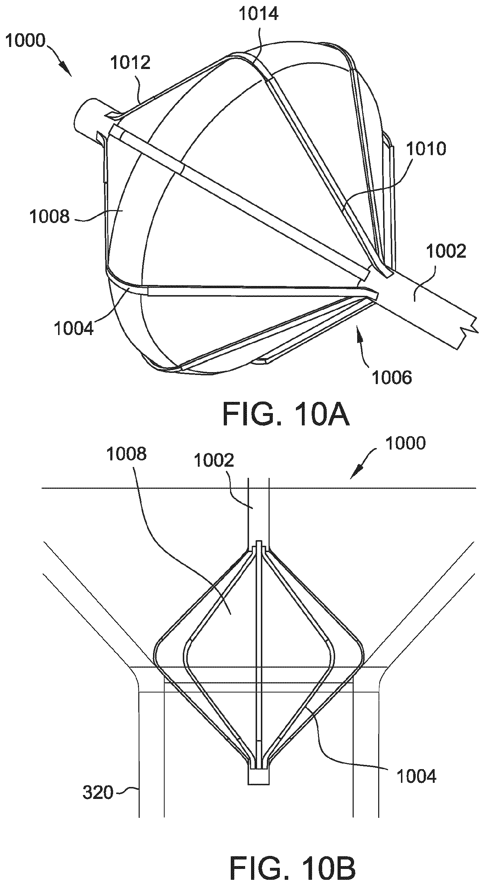

Figure 10A is a perspective view of an alternative embodiment of a catheter assembly 1000, and Figure 10B is a side schematic view of catheter assembly 1000. Catheter assembly 1000 includes a shaft 1002 and a plurality of splines 1004 surrounding a distal portion 1006 of shaft 1002, similar to catheter assembly 900 (shown in Figure 9). However, in contrast to catheter assembly 900, catheter assembly 1000 includes a balloon 1008 enclosed by splines 1004. Balloon 1008 may be selectively inflated to occupy the space between splines 1004. Notably, balloon 1008 functions as an insulator, and generally reduces energy losses relative to catheter assembly 900, which may result in increased lesion size.

Each spline 1004 includes a proximal end 1010 coupled to shaft 1002 and a distal end 1012 coupled to shaft 1002. From proximal end 1010, spline 1004 extends radially outward to an inflection point 1014, and then extends radially inward to distal end 1012. Figure 10B shows catheter assembly 1000 positioned within pulmonary vein 320.

During delivery, splines 1004 and balloon 1008 may be collapsed. To perform ablation, splines 1004 are deployed with inflection points 1014 extending radially outward, and balloon 1008 is selectively inflated to occupy the space between splines 1004.

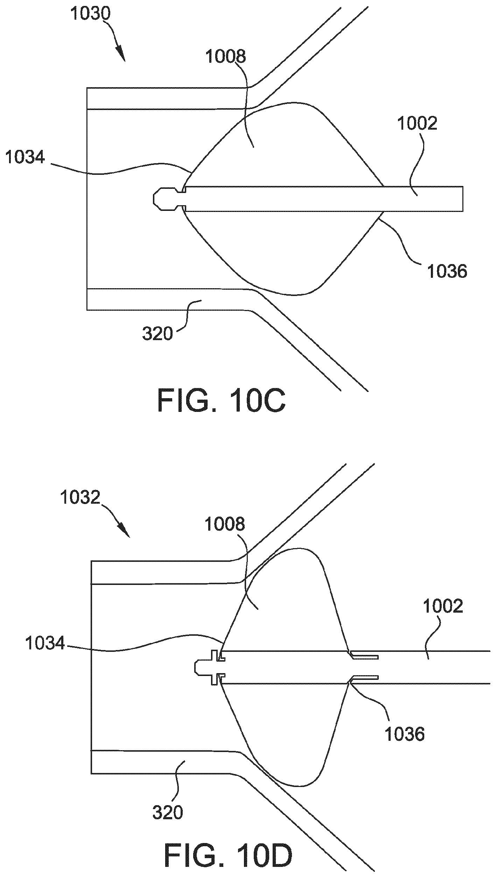

In some embodiments, the shape of balloon 1008 may be selectively changed to improve ablation. For example, Figure 10C is a schematic diagram showing catheter assembly 100 in a first configuration 1030, and Figure 10D is a schematic diagram showing catheter assembly 100 in a second configuration 1032. In second configuration 1032, splines 1004 are axially compressed relative to first configuration 1030, such that the effective diameter of splines 1004 is greater.

To facilitate selectively transitioning balloon catheter assembly 100 between first and second configurations 1030 and 1032, an inner shaft member (not shown in Figures 10A-10D) may be slidably positioned within shaft 1002, with a distal end of inner shaft member coupled to a distal end 1034 of balloon 1008 and splines 1004. When the inner shaft member is pulled proximally relative to shaft 1002, distal end 1034 of balloon 1008 and splines 1004 are also pulled proximally relative to a proximal end 1036 of balloon 1008 and splines 1004, thereby compressing balloon 1008 and splines 1004 axially. The position of the inner shaft member relative to shaft 1002 may be held in place using a suitable locking mechanism (e.g., a Tuohy Borst compression valve), as described in detail herein.

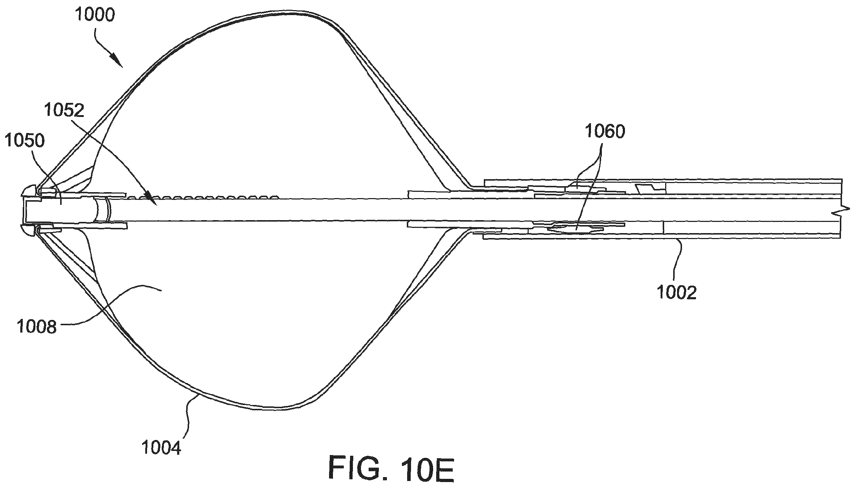

Figure 10E is a side cross-sectional view of catheter assembly 1000. As shown in Figure 10E, catheter assembly 1000 may include one or more magnetic sensors (e.g., to facilitate determining a position and/or orientation of catheter assembly 1000 within a patient). In this embodiment, a hollow-core magnetic sensor 1050 is positioned proximate distal end 1034 of balloon 1008. Hollow-core magnetic sensor 1050 allows for a central lumen 1052 of shaft 1002 to extend therethrough. Hollow-core magnetic sensor 1050 may have, for example, sensing capabilities over five degrees of freedom (enabling detecting position in x, y, and z directions). Central lumen 1052 may be used, for example, to house a guidewire, to house a small diameter mapping catheter, and/or to inject contrast into the patient (e.g., to facilitate determining a position of catheter assembly 1000). Further, in some embodiments, two or more electrodes (not shown) are coupled to shaft 1002 to facilitate impedance-based localization.

Catheter assembly 1000 also includes two solid-core magnetic sensors 1060 positioned proximate proximal end 1032 of balloon 1008. Solid-core magnetic sensors 1060 may be, for example, embedded in shaft 1002, but located outside central lumen 1052. Solid-core magnetic sensors 1060 each individually have sensing capabilities over five degrees of freedom, but combined, have sensing capabilities over six degrees of freedom (enabling detecting position in x, y, and z directions, as well as detecting roll of catheter assembly 1000).

In this embodiment, central lumen 1052 facilitates delivering a fluid to the interior of balloon 1008 to selectively inflate balloon 1008. The fluid may include saline or a mixture of saline and contrast agent. In some embodiments, shaft 1002 may include irrigation holes (not shown) that provide fluid communication between central lumen 1052 and the interior of balloon 1008. Further, in some embodiments, central lumen 1052 enables infusing contrast agent distal of catheter assembly 1000, which may assist a user in assessing blood flow through pulmonary vein 320 and assessing how much catheter assembly 1000 occludes pulmonary vein 320.

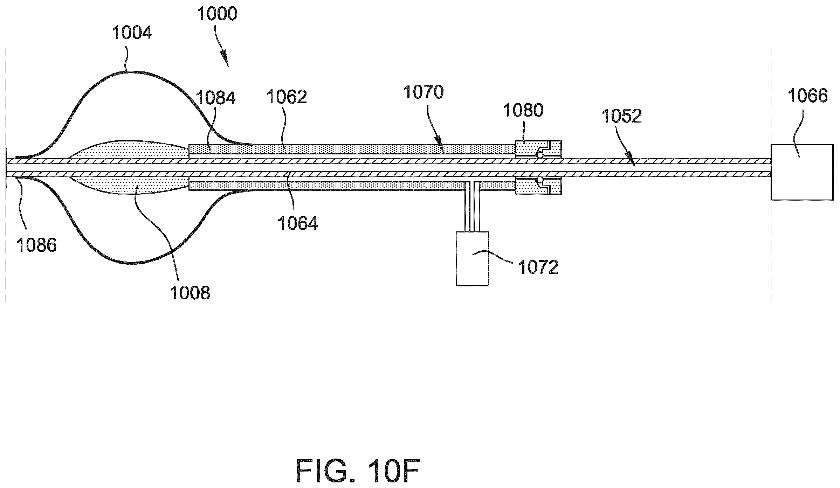

Figures 10F-10J are schematic side cross-sectional views of catheter assembly 1000 illustrating the transitioning of catheter assembly 1000 between different states. As shown in Figure 10F, in this embodiment, shaft 1002 includes an outer shaft element 1062 and an inner shaft element 1064, both of which are tubular components. Inner shaft element 1064 is axially slidable within outer shaft element 1062, and defines central lumen 1052 therethrough. Outer shaft element 1062 may have a French size of, for example, 11.5 French. Alternatively, outer shaft element 1062 may have any suitable dimensions.

A valve 1066 at a distal end of inner shaft element 1064 controls access to central lumen 1052. For example, as noted above, contrast agent may be flushed through central lumen 1052 into the patient to confirm pulmonary vein occlusion. As another example, a mapping catheter (e.g., a 3 French mapping catheter) may extend into the patient through central lumen 1052. As yet another example, a guidewire may extend through central lumen 1052. Those of skill in the art will appreciate that occlusion may be monitored using any suitable technique. For example, pressure monitoring may be used to assess occlusion of the vein, contrast injection may be used to assess occlusion of the vein using fluoroscopy, and/or ultrasound (e.g., Doppler) may be used to assess occlusion.

As shown in Figure 10F, a channel 1070 is defined between outer shaft element 1062 and inner shaft element 1064 in this embodiment. Channel is in fluid communication with an interior of balloon 1008. Further, a stopcock valve 1072 (e.g., a three way stopcock valve) enables inflating and deflating balloon as desired, by controlling fluid flow to the interior of balloon 1008.

In some embodiments, a shape sensing fiber may extend through central lumen 1052 and/or channel 1090. The shape sensing fiber may be a fiber optic fiber that enables a user to precisely determine the position and orientation of the shape sensing fiber, and thus the position and orientation of shaft 1002.

In this embodiment, catheter assembly 1000 further includes a compression valve 1080 that facilitates securing a position of outer shaft element 1062 relative to inner shaft element 1064. Specifically, with compression valve 1080 open, inner shaft element 1064 is slidable relative to outer shaft element 1062. Once inner shaft element 1064 is located at a desired position, compression valve 1080 may be closed to prevent inner shaft element 1064 from sliding relative to outer shaft element 1026. Compression valve 1080 also seals off a proximal end of channel 1070.

As shown in Figure 10F, each spline 1004 extends between a distal end 1084 of outer shaft element 1026 and a distal end 1086 of inner shaft element 1064. Accordingly, by sliding inner shaft element distal end 1086 relative to outer shaft element distal end 1084, the shape of splines 1004 is adjustable.

Figure 10F shows splines 1004 in a neutral position, with balloon 1008 deflated. Specifically, in this embodiment, splines 1004 are made of a shape memory material (e.g., Nitinol) such that splines 1004 assume the shape shown in Figure 10F when not subjected to any external forces or biases.

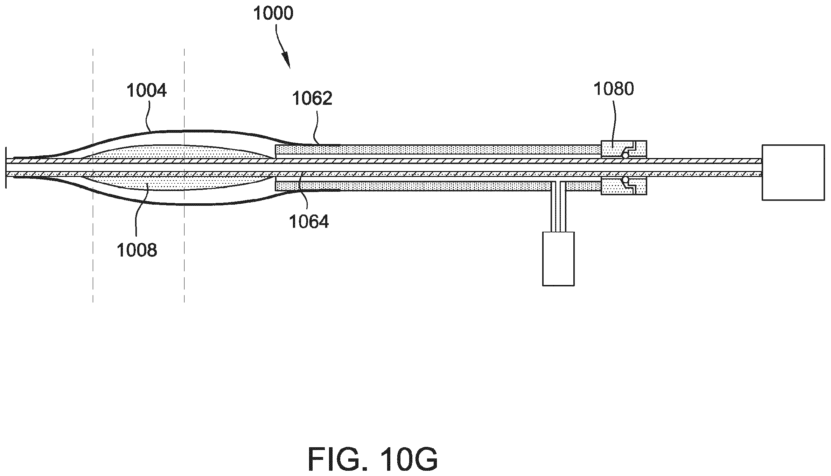

To compress catheter assembly 1000 (e.g., for delivery of catheter assembly 1000), inner shaft element 1064 is slid distally relative to outer shaft element 1062. This causes splines 1004 to collapse inward towards inner shaft element, transitioning catheter assembly 1000 to a collapsed position, as shown in Figure 10G. In Figure 10G, balloon 1008 is deflated. Catheter assembly 1000 may be locked in the collapsed position using compression valve 1080.

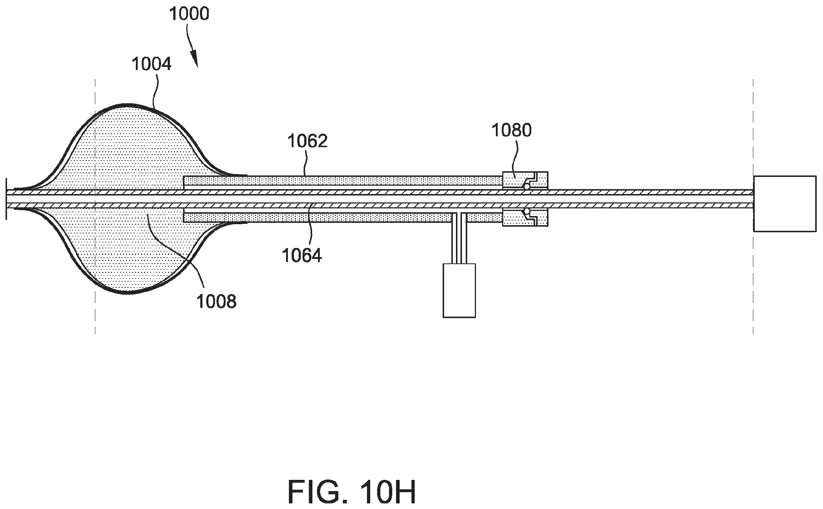

Referring now to Figure 10H, during therapy, balloon 1008 is inflated to fill the space between splines 1004. Specifically, as shown in Figure 10H, with balloon 1008 inflated, balloon 1008 generally conforms to the shape of the basket formed by splines 1004.

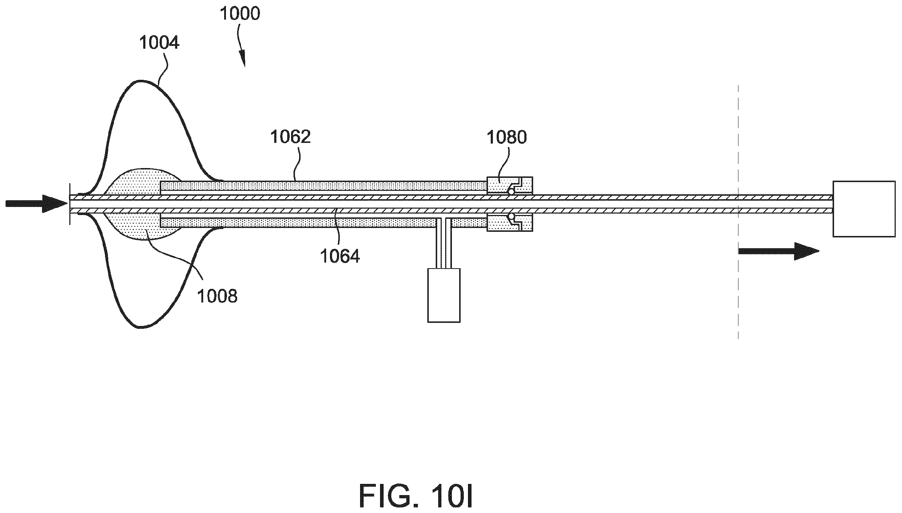

As discussed above in connection with Figures 10C and 10D, the basket formed by splines 1004 may also be compressed to increase an outer diameter of catheter assembly 1000. Specifically, as shown in Figure 10I, when inner shaft element 1064 is slid proximally relative to outer shaft element 1062, this causes splines 1004 to flex outward , transitioning catheter assembly 1000 to a compressed position. In Figure 10I, balloon 1008 is deflated. Catheter assembly 1000 may be locked in the compressed position using compression valve 1080.

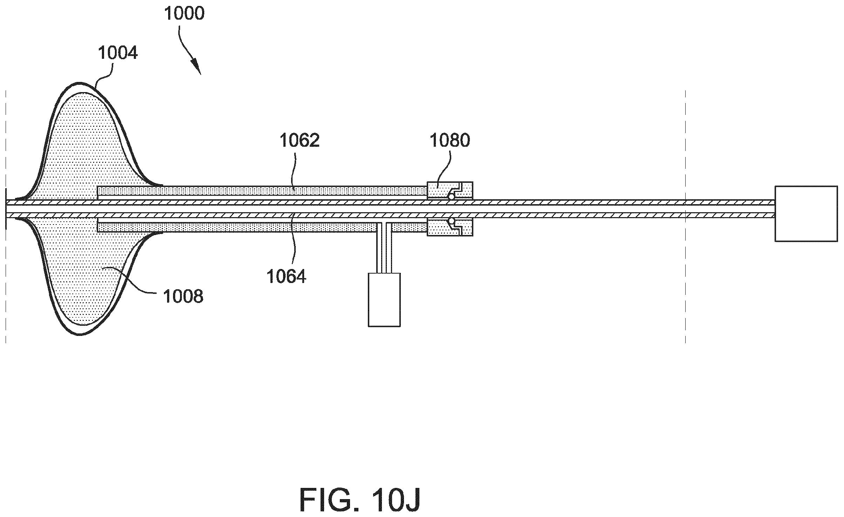

Referring now to Figure 10J, in the compressed position, balloon 1008 may be inflated to fill the space between splines 1004. Specifically, as shown in Figure 10J, with balloon 1008 inflated, balloon 1008 generally conforms to the shape of the basket formed by splines 1004 in the compressed position.

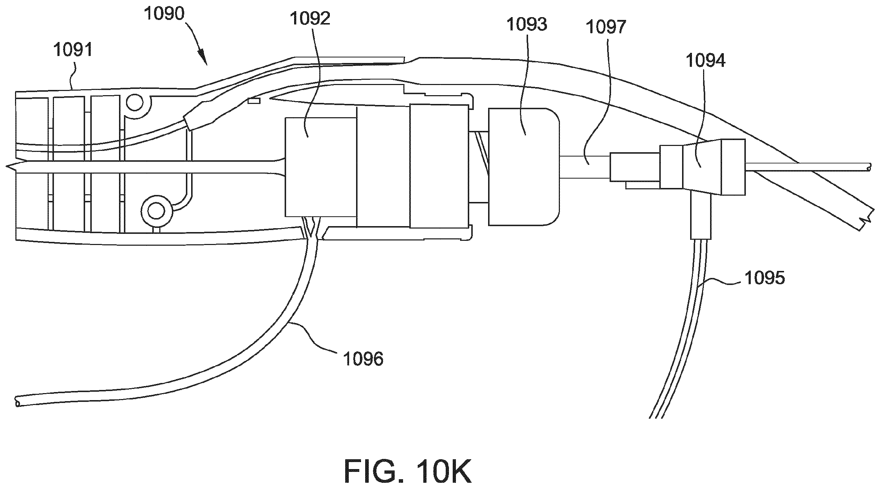

Figure 10K is a side cross-sectional view of a portion of a handle 1090 that may be used with catheter assembly 1000. Handle 1090 includes a housing 1091 and a compression valve component 1092 (e.g., including compression valve 1080) positioned within housing 1091. Further, handle 1090 includes a rotatable knob 1093 coupled to compression valve component 1092 at a distal end of housing 1091. By rotating knob 1093, a user can selectively open and close compression valve 1080 to secure a position of inner shaft element 1064 as desired.

Further, as shown in Figure 10K, a positioning component 1094 is coupled to a distal end of inner shaft element 1064. With compression valve 1080 open, positioning component 1094 may be slid axially relative to housing 1091 to slide inner shaft element 1064 relative to outer shaft element 1062, as described above. A first fluid supply line 1095 (i.e., for supplying fluid to central lumen 1052) and a second fluid supply line 1096 (i.e., for supplying fluid to channel 1070) are also should in Figure 10K.

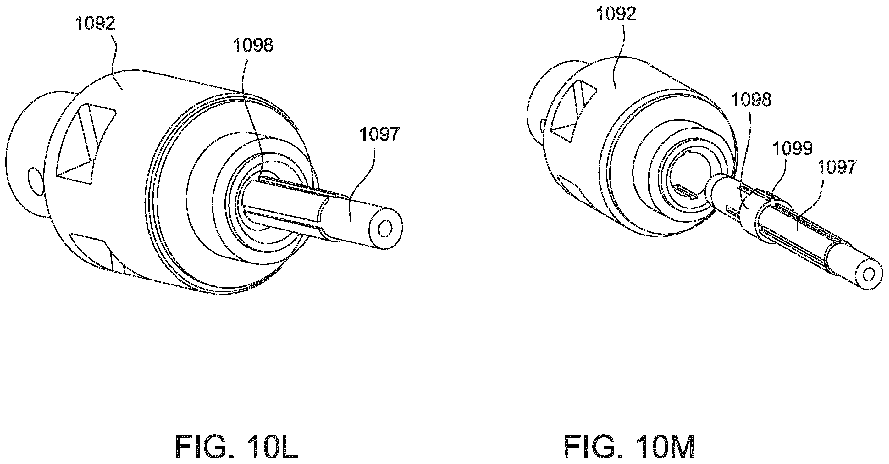

To prevent rotation of positioning component 1094 and first fluid supply line 1095 relative to housing 1091, a pin 1097 non-rotatably couples positioning component 1094 to compression valve component 1092. Specifically, as shown in a perspective view in Figure 10L and in an exploded view in Figure 10M, a ring 1098 couples pin 1097 to compression valve component 1092. Ring 1098 has protrusions 1099 that engage slots defined in compression valve component 1092 and pin 1097 so that pin 1097 (and by extension, positioning component 1094) is not capable of rotation relative to compression valve component 1092.

In some embodiments, one or more lumens (lumen) are defined within outer shaft element 1062 (e.g., between an inner and outer surface of outer shaft element 1062). The lumens may be used to route electrical wires (i.e., for supplying power to splines 1004) and/or activation wires (i.e., for controlling an orientation of catheter assembly 1000) through catheter assembly 1000. For example, in one embodiment, positive electrical wires that supply energy to positive splines 1004 are all routed through a first lumen, and negative electrical wires that supply energy to negative splines are all routed through a second, separate lumen. This effectively isolates the positive electrical wires from the negative electrical wires.

Each electrical wire may be coupled to an associated spline 1004, for example, through a weld, with the weld and proximal ends of the splines arranged on a proximal coupler component (not shown). Further, in some embodiments, a strain relief component (not shown) is coupled to an exterior of the inner shaft element 1064 distal of the outer shaft element 1062, to prevent inner shaft element 1064 from excess bending.

Catheter assembly 1000 provides several advantages. For example, the combination of balloon 1008 and splines 1004 facilitates straightforward delivery and deployment of catheter assembly 1000. Further, balloon 1008 drives more energy into ablated tissue, and stabilizes splines 1004 to prevent lateral movement. In addition, using splines 1004 as electrodes instead of individual smaller electrodes facilitates reducing the cost and increasing the reliability of catheter assembly 1000.

Splines 1004 may all have the same length, or at least some of splines 1004 may have different lengths. Further, insulating material (e.g., a PET or PEBA material) on each spline 1004 may have the same length, or at least some splines 1004 may have insulating material with different lengths. In addition, in some embodiments, catheter assembly 1000 includes a distal electrode (not shown) positioned distal of splines 1004. The distal electrode may be used to perform point ablation (e.g., by creating a bipole between the distal electrode and one of splines 1004), and/or may be used for visualization/mapping purposes (e.g., using the distal electrode in combination with an electrode on shaft 1002).

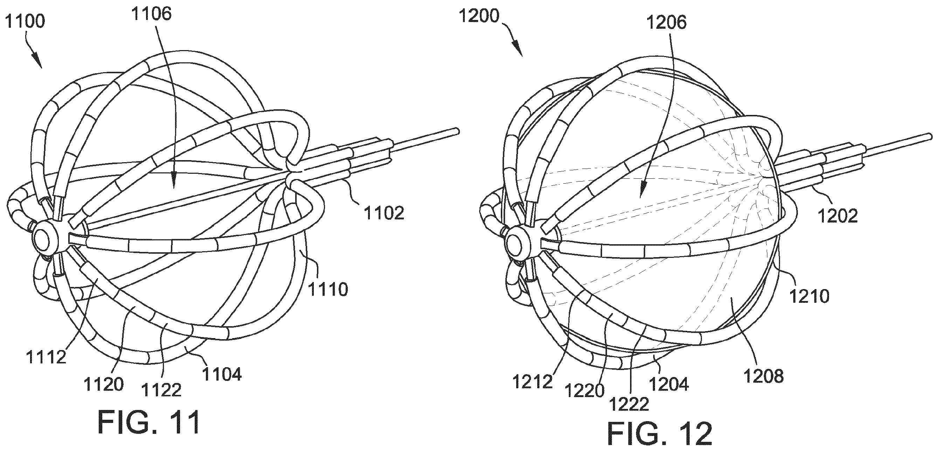

Figure 11 is a perspective view of an alternative embodiment of a catheter assembly 1100. Catheter assembly 1100 includes a shaft 1102 and a plurality of splines 1102 surrounding a distal portion 1106 of shaft 1102. Each spline 1104 includes a proximal end 1110 coupled to shaft 1102 and a distal end 1112 coupled to shaft 1102. From proximal end 1110 to distal end 1112, spline 1104 has an arcuate shape that extends radially outward.

In this embodiment, each spline 1104 includes a plurality of individual electrodes 1120. For example, each spline 1104 may include an elastic material (e.g., Nitinol) covered in a polymer tube 1122, with individual electrodes 1120 attached to an exterior of polymer tube 1122. In the embodiment shown, each spline 1104 includes two electrodes 1120. Further, as shown in Figure 11, electrodes 1120 are generally positioned closer to distal end 1112 than proximal end 1110 to correspond to portions of spline 1104 that will contact pulmonary vein 320.

Alternatively, each spline 1104 may include any suitable number and arrangement of electrodes 1120. For example, in some embodiments, each spline 1104 includes four electrodes 1120.

In this embodiment, alternating splines 1104 alternate polarities. That is, electrodes 1120 on a particular spline 1104 have the same polarity, but electrodes 1120 on a particular spline 1104 have a different polarity than electrodes 1120 on adjacent splines 1104. Alternatively, any suitable polarization scheme may be used. During delivery, splines 1104 may be collapsed in towards shaft 1102. Subsequently, to perform ablation, splines 1104 are deployed to extend radially outward.

Including multiple electrodes 1120 on each spline 1104, instead of the elastic material of splines 1104 themselves being electrodes 1120, improves the ability of catheter assembly 1100 to perform various mapping routines, as each electrode 1120 can be used as an individual sensor for acquiring mapping data.

Splines 1104 may all have the same length, or at least some of splines 1004 may have different lengths. Further, insulating material (e.g., a PET or PEBA material) on each spline 1104 may have the same length, or at least some splines 1104 may have insulating material with different lengths. In addition, in some embodiments, catheter assembly 1100 includes a distal electrode (not shown) positioned distal of splines 1104. The distal electrode may be used to perform point ablation (e.g., by creating a bipole between the distal electrode and one of splines 1104), and/or may be used for visualization/mapping purposes (e.g., using the distal electrode in combination with an electrode on shaft 1102).

Figure 12 is a perspective view of an alternative embodiment of a catheter assembly 1200. Catheter assembly 1200 includes a shaft 1202 and a plurality of splines 1204 surrounding a distal portion 1206 of shaft 1202, similar to catheter assembly 1100 (shown in Figure 11). However, in contrast to catheter assembly 1100, catheter assembly 1200 includes a balloon 1208 enclosed by splines 1204. Balloon 1208 may be selectively inflated to fill the space between splines 1204. Notably, balloon 1208 functions as an insulator, and generally reduces energy losses relative to catheter assembly 1200, which may result in increased lesion size.

Each spline 1204 includes a proximal end 1210 coupled to shaft 1202 and a distal end 1212 coupled to shaft 1202. From proximal end 1210 to distal end 1212, spline 1204 has an arcuate shape that extends radially outward.

In this embodiment, each spline 1204 includes a plurality of individual electrodes 1220. For example, each spline 1204 may include an elastic material (e.g., Nitinol) covered in a polymer tube 1222, with individual electrodes 1220 attached to an exterior of polymer tube 1222. In the embodiment shown, each spline 1204 includes two electrodes 1220. Further, as shown in Figure 12, electrodes 1220 are generally positioned closer to distal end 1212 than proximal end 1210 to correspond to portions of spline 1204 that will contact pulmonary vein 320.

Alternatively, each spline 1204 may include any suitable number and arrangement of electrodes 1220. For example, in some embodiments, each spline 1204 includes four electrodes 1220.

In this embodiment, alternating splines 1204 alternate polarities. That is, electrodes 1220 on a particular spline 1204 have the same polarity, but electrodes 1220 on a particular spline 1204 have a different polarity than electrodes 1220 on adjacent splines 1204. Alternatively, any suitable polarization scheme may be used. During delivery, splines 1204 may be collapsed in towards shaft 1202. Subsequently, to perform ablation, splines 1204 are deployed to extend radially outward.

Splines 1204 may all have the same length, or at least some of splines 1204 may have different lengths. Further, insulating material (e.g., a PET or PEBA material) on each spline 1204 may have the same length, or at least some splines 1204 may have insulating material with different lengths. In addition, in some embodiments, catheter assembly 1200 includes a distal electrode (not shown) positioned distal of splines 1204. The distal electrode may be used to perform point ablation (e.g., by creating a bipole between the distal electrode and one of splines 1204), and/or may be used for visualization/mapping purposes (e.g., using the distal electrode in combination with an electrode on shaft 1202).

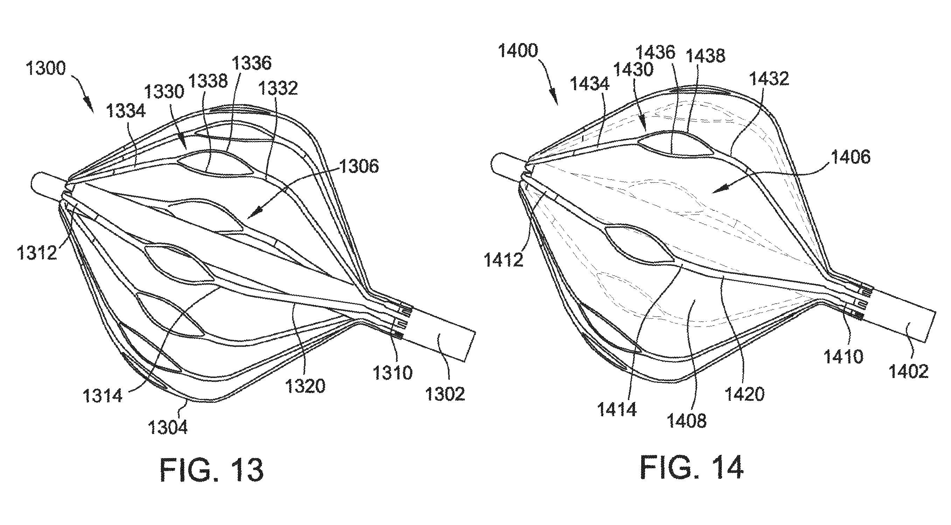

Figure 13 is a perspective view of an alternative embodiment of a catheter assembly 1300. Catheter assembly 1300 includes a shaft 1302 and a plurality of splines 1304 surrounding a distal portion 1306 of shaft 1302. Each spline 1304 includes a proximal end 1310 coupled to shaft 1302 and a distal end 1312 coupled to shaft 1302. From proximal end 1310, spline 1304 extends radially outward to an inflection point 1314, and then extends radially inward to distal end 1312.

Splines 1304 are made of an elastic material (e.g., Nitinol), and function as relatively large electrodes. In this embodiment, alternating splines 1304 alternate polarities. That is, each positive spline 1304 is positioned between two negative splines 1304 and vice-versa. Alternatively, any suitable polarization scheme may be used.

To control the ablation zone of each spline 1304, portions of each spline 1304 may be covered with insulating material 1320 (e.g., heat-shrink or polymer tubing, such as PET or PEBA), and the exposed portions of splines 1304 function as electrodes. In the embodiment shown in Figure 13, inflection point 1314 and portions of spline 1304 between inflection point 1314 and distal end 1312 are generally exposed, while portions of spline 1304 between inflection point 1314 and proximal end 1310 are generally insulated. This results in the portions of spline 1304 that contact pulmonary vein 320 being exposed. Alternatively, any suitable insulation configuration may be used.

As shown in Figure 13, each spline 1304 includes a spread member 1330. Spread member 1330 includes a first end 1332, a second end 1334, a first branch 1336, and a second branch 1338. From first end 1332, first and second branches 1336 and 1338 extend away from one another, before they extend back towards one another and rejoin at second end 1334.

Spread member 1330 may be fabricated, for example, using laser cutting. In this embodiment, all of spread member 1330 is exposed (i.e., not covered with insulating material). Alternatively, portions of spread member 1330 may be covered with insulating material.

Including spread members 1330 on splines 1304 reduces the extent of circumferential gaps between splines 1304, which facilitates increasing lesion volume. Further, additional splines 1304 may be included to further reduce the extent of the circumferential gaps.

In some embodiments, at least one of splines 1304 includes multiple spread members 1330, instead of a single spread member 1330. Further, as shown in Figure 13, spread members 1330 are longitudinally aligned with one another in catheter assembly 1300. However, in some embodiments, at least some spread members 1330 are longitudinally offset relative to one another.

Further, although spread members 1330 are shown as having two branches 1336 and 1338 with substantially equal lengths, spread members 1330 may have different numbers of branches and/or branches with unequal lengths. Further, in some embodiments, a spread member 1330 may be formed from two separates splines 1304, instead of a single spline 1304 forming spread member 1330.

During delivery, splines 1304 may be collapsed inwards towards shaft 1302. Further, when splines 1304 collapse, first and second branches 1336 and 1338 of each spread member 1330 also collapse inwards towards one another, reducing the overall profile of catheter assembly 1300. Subsequently, to perform ablation, splines 1304 are deployed with inflection points 1314 extending radially outward.

Splines 1304 may all have the same length, or at least some of splines 1304 may have different lengths. Further, insulating material on each spline 1304 may have the same length, or at least some splines 1304 may have insulating material with different lengths. In addition, in some embodiments, catheter assembly 1300 includes a distal electrode (not shown) positioned distal of splines 1304. The distal electrode may be used to perform point ablation (e.g., by creating a bipole between the distal electrode and one of splines 1304), and/or may be used for visualization/mapping purposes (e.g., using the distal electrode in combination with an electrode on shaft 1302).

Figure 14 is a perspective view of an alternative embodiment of a catheter assembly 1400. Catheter assembly 1400 includes a shaft 1402 and a plurality of splines 1404 surrounding a distal portion 1406 of shaft 1402. However, in contrast to catheter assembly 1300 (shown in Figure 13), catheter assembly 1400 includes a balloon 1408 enclosed by splines 1404. Balloon 1408 may be selectively inflated to fill the space between splines 1404. Notably, balloon 1408 functions as an insulator, and generally reduces energy losses relative to catheter assembly 1300, which may result in increased lesion size.

Each spline 1404 includes a proximal end 1410 coupled to shaft 1402 and a distal end 1412 coupled to shaft 1402. From proximal end 1410, spline 1404 extends radially outward to an inflection point 1414, and then extends radially inward to distal end 1412.

Splines 1404 are made of an elastic material (e.g., Nitinol), and function as relatively large electrodes. In this embodiment, alternating splines 1404 alternate polarities. That is, each positive spline 1404 is positioned between two negative splines 1404 and vice-versa. Alternatively, any suitable polarization scheme may be used.

To control the ablation zone of each spline 1404, portions of each spline 1404 may be covered with insulating material 1420 (e.g., heat-shrink or polymer tubing, such as PET or PEBA), and the exposed portions of splines 1404 function as electrodes. In the embodiment shown in Figure 14, inflection point 1414 and portions of spline 1404 between inflection point 1414 and distal end 1412 are generally exposed, while portions of spline 1404 between inflection point 1414 and proximal end 1410 are generally insulated. This results in the portions of spline 1404 that contact pulmonary vein 320 being exposed. Alternatively, any suitable insulation configuration may be used.

As shown in Figure 14, each spline 1404 includes a spread member 1430. Spread member 1430 includes a first end 1432, a second end 1434, a first branch 1436, and a second branch 1438. From first end 1432, first and second branches 1436 and 1438 extend away from one another, before they extend back towards one another and rejoin at second end 1434.

Spread member 1430 may be fabricated, for example, using laser cutting. In this embodiment, all of spread member 1430 is exposed (i.e., not covered with insulating material). Alternatively, portions of spread member 1430 may be covered with insulating material.

Including spread members 1430 on splines 1404 reduces the extent of circumferential gaps between splines 1404, which facilitates increasing lesion volume. Further, additional splines 1404 may be included to further reduce the extent of the circumferential gaps.

In some embodiments, at least one of splines 1404 includes multiple spread members 1430, instead of a single spread member 1430. Further, as shown in Figure 14, spread members 1430 are longitudinally aligned with one another in catheter assembly 1400. However, in some embodiments, at least some spread members 1430 are longitudinally offset relative to one another.

During delivery, splines 1404 may be collapsed inwards towards shaft 1402. Further, when splines 1404 collapse, first and second branches 1436 and 1438 of each spread member 1430 also collapse inwards towards one another, reducing the overall profile of catheter assembly 1400. Subsequently, to perform ablation, splines 1404 are deployed with inflection points 1414 extending radially outward.

Splines 1404 may all have the same length, or at least some of splines 1404 may have different lengths. Further, insulating material on each spline 1404 may have the same length, or at least some splines 1404 may have insulating material with different lengths. In addition, in some embodiments, catheter assembly 1400 includes a distal electrode (not shown) positioned distal of splines 1404. The distal electrode may be used to perform point ablation (e.g., by creating a bipole between the distal electrode and one of splines 1404), and/or may be used for visualization/mapping purposes (e.g., using the distal electrode in combination with an electrode on shaft 1402).

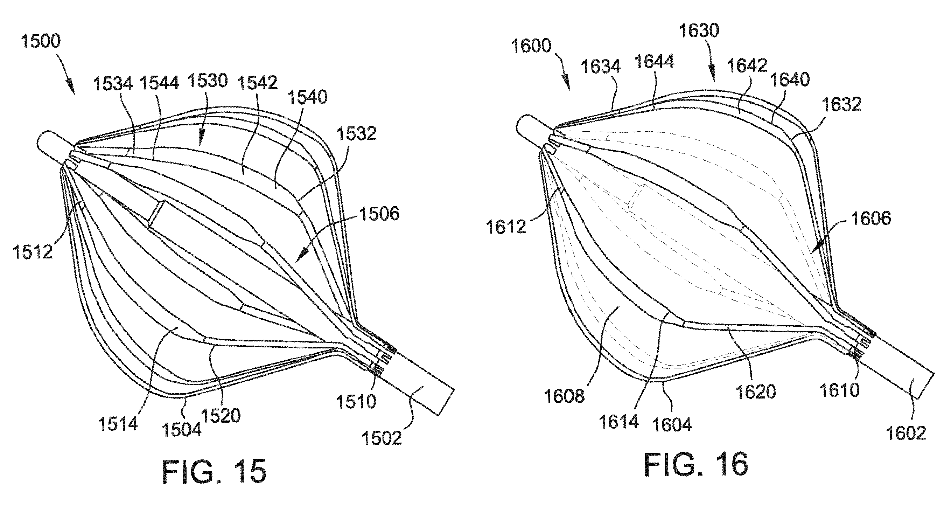

Figure 15 is a perspective view of an alternative embodiment of a catheter assembly 1500. Catheter assembly 1500 includes a shaft 1502 and a plurality of splines 1504 surrounding a distal portion 1506 of shaft 1502. Each spline 1504 includes a proximal end 1510 coupled to shaft 1502 and a distal end 1512 coupled to shaft 1502. From proximal end 1510, spline 1504 extends radially outward to an inflection point 1514, and then extends radially inward to distal end 1512.

Splines 1504 are made of an elastic material (e.g., Nitinol), and function as relatively large electrodes. In this embodiment, alternating splines 1504 alternate polarities. That is, each positive spline 1504 is positioned between two negative splines 1504 and vice-versa. Alternatively, any suitable polarization scheme may be used.

To control the ablation zone of each spline 1504, portions of each spline 1504 may be covered with insulating material 1520 (e.g., heat-shrink or polymer tubing, such as PET or PEBA), and the exposed portions of splines 1504 function as electrodes. In the embodiment shown in Figure 15, inflection point 1514 and portions of spline 1504 between inflection point 1514 and distal end 1512 are generally exposed, while portions of spline 1504 between inflection point 1514 and proximal end 1510 are generally insulated. This results in the portions of spline 1504 that contact pulmonary vein 320 being exposed. Alternatively, any suitable insulation configuration may be used.

As shown in Figure 15, each spline 1504 includes a tapered member 1530. Tapered member 1530 includes a first end 1532 and a second end 1534. From first end 1532, a width of tapered member 1530 tapers outward along a first tapered portion 1540, the width stays substantially constant along a middle portion 1542, and the width tapers back inward along a second tapered portion 1544 towards second end 1534. In some embodiments, tapered member 1530 includes only a single tapered portion.

Tapered member 1530 may be fabricated, for example, using laser cutting. In this embodiment, all of tapered member 1530 is exposed (i.e., not covered with insulating material). Alternatively, portions of tapered member 1530 may be covered with insulating material. Further in this embodiment, tapered member 1530 functions as a single electrode. Alternatively, tapered member 1530 may be split into multiple individual electrodes (e.g., including insulating material and ring electrodes as appropriate).

Including tapered members 1530 on splines 1504 reduces the extent of circumferential gaps between splines 1504, which facilitates increasing lesion volume. Further, additional splines 1504 may be included to further reduce the extent of the circumferential gaps.

In some embodiments, at least one of splines 1504 includes multiple tapered members 1530, instead of a single tapered member 1530. Further, as shown in Figure 15, tapered members 1530 are longitudinally aligned with one another in catheter assembly 1500. However, in some embodiments, at least some tapered members 1530 are longitudinally offset relative to one another.

During delivery, splines 1504 may be collapsed inwards towards shaft 1502. Subsequently, to perform ablation, splines 1504 are deployed with inflection points 1514 extending radially outward.

Splines 1504 may all have the same length, or at least some of splines 1504 may have different lengths. Further, insulating material on each spline 1504 may have the same length, or at least some splines 1504 may have insulating material with different lengths. In addition, in some embodiments, catheter assembly 1500 includes a distal electrode (not shown) positioned distal of splines 1504. The distal electrode may be used to perform point ablation (e.g., by creating a bipole between the distal electrode and one of splines 1504), and/or may be used for visualization/mapping purposes (e.g., using the distal electrode in combination with an electrode on shaft 1502).

Figure 16 is a perspective view of an alternative embodiment of a catheter assembly 1600. Catheter assembly 1600 includes a shaft 1602 and a plurality of splines 1604 surrounding a distal portion 1606 of shaft 1602. However, in contrast to catheter assembly 1500 (shown in Figure 15), catheter assembly 1600 includes a balloon 1608 enclosed by splines 1604. Balloon 1608 may be selectively inflated to fill the space between splines 1604. Notably, balloon 1608 functions as an insulator, and generally reduces energy losses relative to catheter assembly 1500, which may result in increased lesion size.

Each spline 1604 includes a proximal end 1610 coupled to shaft 1602 and a distal end 1612 coupled to shaft 1602. From proximal end 1610, spline 1604 extends radially outward to an inflection point 1614, and then extends radially inward to distal end 1612.

Splines 1604 are made of an elastic material (e.g., Nitinol), and function as relatively large electrodes. In this embodiment, alternating splines 1604 alternate polarities. That is, each positive spline 1604 is positioned between two negative splines 1604 and vice-versa. Alternatively, any suitable polarization scheme may be used.

To control the ablation zone of each spline 1604, portions of each spline 1604 may be covered with insulating material 1620 (e.g., heat-shrink or polymer tubing, such as PET or PEBA), and the exposed portions of splines 1604 function as electrodes. In the embodiment shown in Figure 16, inflection point 1614 and portions of spline 1604 between inflection point 1614 and distal end 1612 are generally exposed, while portions of spline 1604 between inflection point 1614 and proximal end 1610 are generally insulated. This results in the portions of spline 1604 that contact pulmonary vein 320 being exposed. Alternatively, any suitable insulation configuration may be used.

As shown in Figure 16, each spline 1604 includes a tapered member 1630. Tapered member 1630 includes a first end 1632 and a second end 1634. From first end 1632, a width of tapered member 1630 tapers outward along a first tapered portion 1640, the width stays substantially constant along a middle portion 1642, and the width tapers back inward along a second tapered portion 1644 towards second end 1634. In some embodiments, tapered member 1530 includes only a single tapered portion.

Tapered member 1630 may be fabricated, for example, using laser cutting. In this embodiment, all of tapered member 1630 is exposed (i.e., not covered with insulating material). Alternatively, portions of tapered member 1630 may be covered with insulating material. Further in this embodiment, tapered member 1630 functions as a single electrode. Alternatively, tapered member 1630 may be split into multiple individual electrodes (e.g., including insulating material and ring electrodes as appropriate).

Including tapered members 1630 on splines 1604 reduces the extent of circumferential gaps between splines 1604, which facilitates increasing lesion volume. Further, additional splines 1604 may be included to further reduce the extent of the circumferential gaps.

In some embodiments, at least one of splines 1604 includes multiple tapered members 1630, instead of a single tapered member 1630. Further, as shown in Figure 16, tapered members 1630 are longitudinally aligned with one another in catheter assembly 1600. However, in some embodiments, at least some tapered members 1630 are longitudinally offset relative to one another.

During delivery, splines 1604 may be collapsed inwards towards shaft 1602. Subsequently, to perform ablation, splines 1604 are deployed with inflection points 1614 extending radially outward.

Splines 1604 may all have the same length, or at least some of splines 1604 may have different lengths. Further, insulating material on each spline 1604 may have the same length, or at least some splines 1604 may have insulating material with different lengths. In addition, in some embodiments, catheter assembly 1600 includes a distal electrode (not shown) positioned distal of splines 1604. The distal electrode may be used to perform point ablation (e.g., by creating a bipole between the distal electrode and one of splines 1604), and/or may be used for visualization/mapping purposes (e.g., using the distal electrode in combination with an electrode on shaft 1602).

Due at least in part to the length of the exposed portions of the splines, the catheter assemblies described herein may, in some embodiments, create lesions having a length in a range from approximately 1.0 to 1.5 centimeters. This results in a wide band of lesions to help prevent breakthrough, as opposed to lesions in at least some known systems (which may have a length of approximately 4 or 5 mm). This is important as lesions are often not homogenous. Further, the larger lesion length may result in ablating a large area of the PV antrum. This is advantageous, as the PV antrum includes many transition tissue fibers that may be pro-arrhythmic.

In the embodiments described herein, the splines are generally straight. However, splines may have any suitable shape. For example, in some embodiments, splines may have a sigmoidal shape, which may facilitate relieving stresses when the splines are expanded and contracted.

In addition, although the embodiments described herein are shown with a particular number of splines, those of skill in the art will appreciate that any suitable number of splines may be included. For example, catheter assemblies may include four, six, eight, ten, twelve, fourteen, sixteen, eighteen, or twenty splines in some embodiments.

As noted above, in catheter assemblies that include a balloon enclosed by the splines (and corresponding electrodes), the balloon functions as an insulator, and generally reduces energy losses. This occurs because the balloon causes the electrodes to primarily transfer energy outward, away from the balloon, resulting in the energy predominantly being transferred to target tissue instead of into the blood pool.

During PFA therapy, at least some known systems may generate microbubbles, which may be undesirable. However, it has been observed that increased current density at electrodes results in increased microbubble formation. Accordingly, reduced current density generally results in reduced microbubble formation. In at least some of the embodiments described herein, the electrodes have a relatively large surface area (e.g., in embodiments where a relatively long, exposed portion of the spline functions as the electrode). This larger surface area results in lower current density, and thus reduced microbubble formation (in addition to being more efficient at delivering energy to target tissue as discussed above).

Those of skill in the art will appreciate that the various embodiments of catheter assemblies may be implemented independently from one another or in any suitable combination.

Further, the catheter assemblies described herein may have any suitable dimensions. For example, in some embodiments, with the splines in an extended configuration, the catheter assemblies described herein have a diameter in a range of about 28-35 mm. Alternatively, the catheter assemblies described herein may have a smaller diameter (e.g., in a range of about 8-10 mm).

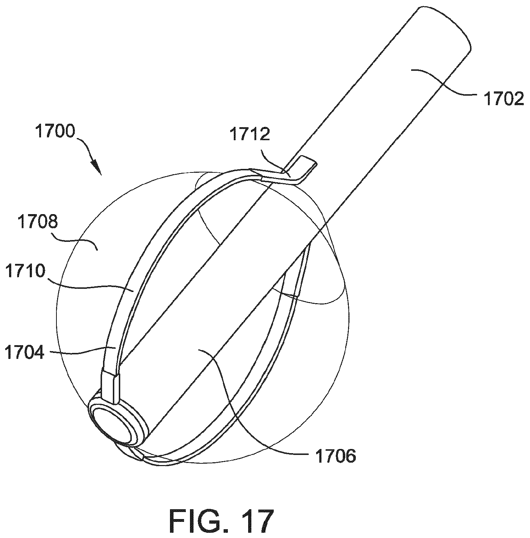

For example, Figure 17 is a perspective view of an alternative embodiment of a catheter assembly 1700. Catheter assembly 1700 includes a shaft 1702 and a plurality of splines 1704 surrounding a distal portion 1706 of shaft 1702. Catheter assembly 1700 also includes a balloon 1708 enclosed by splines 1704. Balloon 1708 may be selectively inflated to fill the space between splines 1704. Notably, balloon 1708 functions as an insulator, and generally reduces energy losses, which may result in increased lesion size.

Relative to catheter assemblies 300, 400, 500, 600, 900, 1000, 1100, 1200, 1300, 1400, 1500, and 1600, catheter assembly 1700 may be smaller, with a more simplified design, which may result in reduced manufacturing costs. For example, in some embodiments, catheter assemblies 300, 400, 500, 600, 900, 1000, 1100, 1200, 1300, 1400, 1500, and 1600 may have a diameter in a range of about 28-35 mm with the splines expanded, in order to facilitate performing PVI. In contrast, with splines 1704 expanded, catheter assembly 1700 may have a diameter in a range of about 8-10 mm (e.g., a diameter of 9 mm). Further, when collapsed, catheter assembly 1700 may be deliverable using a 7.5 French shaft.

The smaller diameter allows catheter assembly 1700 to generate smaller, more focused lesions. For example, catheter assembly 1700 may be used to treat gaps that remain after one of catheter assemblies 300, 400, 500, 600, 900, 1000, 1100, 1200, 1300, 1400, 1500, and 1600 has been used to perform PVI. Accordingly, catheter assembly 1700 may be used to supplement procedures performed using catheter assemblies 300, 400, 500, 600, 900, 1000, 1100, 1200, 1300, 1400, 1500, and 1600. As another example, catheter assembly 1700 may be used to treat other targets, such as the posterior wall.

From simulations, it has been demonstrated that catheter assembly 1700 generates lesions substantially consistently, regardless of the orientation of catheter assembly 1700 to target tissue. For example, in simulations, lesions having a depth of at least 4 mm were achieved with shaft 1702 at angles of approximately 10°, 45°, and 90° relative to a surface of the target tissue.

In the embodiment shown, each spline 1704 includes an exposed portion 1710 that functions as an electrode, and an insulated portion 1712 proximal of exposed portion 1710. In some embodiments, another insulated portion 1712 is included distal of exposed portion 1710. Alternatively, this distal insulated portion 1712 may be omitted. Although splines 1704 are shows as having a constant width, those of skill in the art will appreciated that other spline shapes may be used (e.g., tapered shapes, split shapes, etc.).

To perform ablation, a voltage (e.g., 1500 V) is applied to catheter assembly 1700. Specifically, the voltage may be applied between splines 1704 in accordance with a bipolar approach. Alternatively, the voltage may be applied between one or more splines 1704 and a separate electrode (e.g., a body patch electrode) under a monopolar approach. Further, voltages may be applied to splines 1704 concurrently, or sequentially (e.g., via multiplexing).

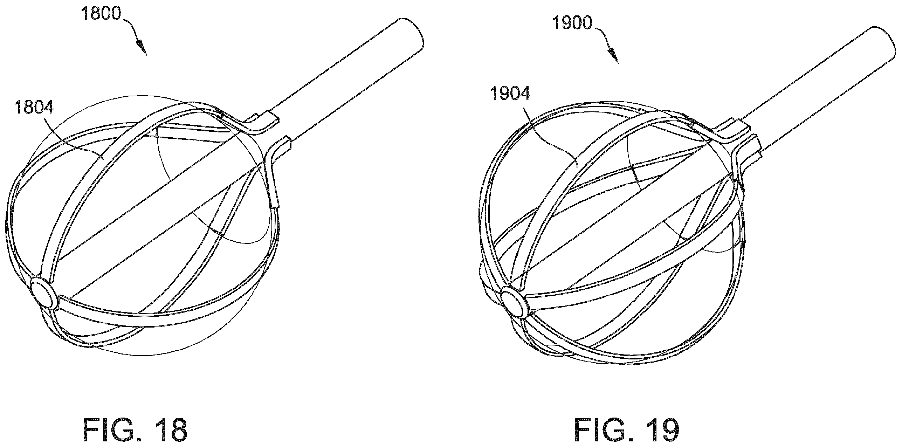

Catheter assembly 1700 includes two splines 1704. Alternatively, more splines may be included. For example, Figure 18 is a perspective view of an alternative embodiment of a catheter assembly 1800 that includes four splines 1804, and Figure 19 is a perspective view of an alternative embodiment of a catheter assembly 1900 that includes six splines 1904. Aside from the number of splines, catheter assemblies 1800 and 1900 are substantially similar to catheter assembly 1700.

The embodiments described herein provide systems and methods for electroporation catheters are described herein. An example electroporation catheter includes a shaft, and a plurality of splines forming a basket around a distal portion of the shaft, each spline extending between a proximal end that is coupled to the shaft and a distal end that is coupled to the shaft, wherein each spline of the plurality of splines comprises at least one energizable electrode. The electroporation catheter further includes a balloon positioned within the basket formed by the plurality of splines, the balloon selectively inflatable to facilitate securing a position of the plurality of splines.

Although certain embodiments of this disclosure have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions without departing from the scope of the disclosure, it is intended that all matter contained in the above description or shown in the accompanying drawings shall be interpreted as illustrative and not in a limiting sense.

The present application also includes the following numbered clauses::
1. An electroporation catheter comprising:
   a shaft;
   a plurality of splines forming a basket around a distal portion of the shaft, each spline extending between a proximal end that is coupled to the shaft and a distal end that is coupled to the shaft, wherein each spline of the plurality of splines comprises at least one energizable electrode; and
   a balloon positioned within the basket formed by the plurality of splines, the balloon selectively inflatable to facilitate securing a position of the plurality of splines.
2.The electroporation catheter in accordance with clause 1, wherein each spline comprises:
   a body comprising an elastic conductive material; and
   an insulating material covering portions of the body, wherein at least one exposed portion of the body corresponds to the at least one selectively energizable electrode.
3. The electroporation catheter in accordance with clause 1, wherein each spline comprises at least one spread member corresponding to the at least one electrode, the at least one spread member comprising:
   a first end;
   a second end;
   a first branch extending from the first end to the second end; and
   a second branch extending from the first end to the second end, the first and second branches spaced apart from one another.
4. The electroporation catheter in accordance with clause 1, wherein each spline comprises at least one tapered member corresponding to the at least one electrode, the at least one tapered member comprising:
   a first end;
   a second end; and
   at least one tapered portion positioned between the first end and the second end.
5. The electroporation catheter in accordance with clause 4, wherein the at least one
   tapered portion comprises a first tapered portion and a second tapered portion, and wherein the at least one tapered member further comprises a middle portion extending between the first tapered portion and the second tapered portion, the middle portion having a constant width.
6. The electroporation catheter in accordance with clause 1, wherein the shaft comprises:
   an outer shaft element; and
   an inner shaft element extending through the outer shaft element, wherein the distal end of each spline is operatively connected to a distal end of the inner shaft element, wherein the proximal end of each spline is connected to a distal end of the outer shaft element, and wherein the inner shaft element is slidable relative to the outer shaft element to adjust an effective diameter of the basket formed by the plurality of splines.
7. The electroporation catheter in accordance with clause 6, wherein sliding the inner shaft element distally relative to the shaft element decreases the effective diameter, and wherein sliding the inner shaft element proximally relative to the shaft element increases the effective diameter.
8. The electroporation catheter in accordance with clause 6, wherein the inner shaft element defines a central lumen, the central lumen configured to receive at least one of contrast agent, a mapping catheter, a guidewire, and a shape sensing fiber.
9. The electroporation catheter in accordance with clause 6, wherein a channel is defined between the inner shaft element and the outer shaft element, the channel in fluid communication with an interior of the balloon to facilitate selectively inflating the balloon using a fluid.
10. The electroporation catheter in accordance with clause 1, wherein alternating splines have electrodes with alternating polarities.
11. An electroporation system comprising:
   a generator; and
   a catheter coupled to the generator, the catheter comprising:
      a handle;
      a shaft extending distally from the handle;
      a plurality of splines forming a basket around a distal portion of the shaft, each spline extending between a proximal end that is coupled to the shaft and a distal end that is coupled to the shaft, wherein each spline of the plurality of splines comprises at least one energizable electrode; and
      a balloon positioned within the basket formed by the plurality of splines, the balloon selectively inflatable to facilitate securing a position of the plurality of splines.
12. The electroporation system in accordance with clause 11, wherein each spline comprises:
   a body comprising an elastic conductive material; and
   an insulating material covering portions of the body, wherein at least one exposed portion of the body corresponds to the at least one selectively energizable electrode.
13. The electroporation system in accordance with clause 11, wherein each spline comprises at least one spread member corresponding to the at least one electrode, the at least one spread member comprising:
   a first end;
   a second end;
   a first branch extending from the first end to the second end; and
   a second branch extending from the first end to the second end, the first and second branches spaced apart from one another.
14. The electroporation system in accordance with clause 11, wherein each spline comprises at least one tapered member corresponding to the at least one electrode, the at least one tapered member comprising:
   a first end;
   a second end; and
   at least one tapered portion positioned between the first end and the second end.
15. The electroporation system in accordance with clause 14, wherein the at least one tapered portion comprises a first tapered portion and a second tapered portion, and wherein the at least one tapered member further comprises a middle portion extending between the first tapered portion and the second tapered portion, the middle portion having a constant width.
16. The electroporation system in accordance with clause 11, wherein the shaft compnses:
   an outer shaft element; and
   an inner shaft element extending through the outer shaft element, wherein the distal end of each spline is operatively connected to a distal end of the inner shaft element, wherein the proximal end of each spline is connected to a distal end of the outer shaft element, and wherein the inner shaft element is slidable relative to the outer shaft element to adjust an effective diameter of the basket formed by the plurality of splines.
17. The electroporation system in accordance with clause 16, wherein sliding the inner shaft element distally relative to the shaft element decreases the effective diameter, and wherein sliding the inner shaft element proximally relative to the shaft element increases the effective diameter.
18. The electroporation system in accordance with clause 16, wherein the inner shaft element defines a central lumen, the central lumen configured to receive at least one of contrast agent, a mapping catheter, a guidewire, and a shape sensing fiber.
19. The electroporation system in accordance with clause 16, wherein a channel is defined between the inner shaft element and the outer shaft element, the channel in fluid communication with an interior of the balloon to facilitate selectively inflating the balloon using a fluid.
20. A method of assembling an electroporation catheter, the method comprising:
   providing a shaft;
   coupling a plurality of splines to the shaft to form a basket around a distal portion of the shaft, each spline extending between a proximal end that is coupled to the shaft and a distal end that is coupled to the shaft, wherein each spline of the plurality of splines includes at least one energizable electrode; and
   positioning a balloon within the basket formed by the plurality of splines, the balloon selectively inflatable to facilitate securing a position of the plurality of splines.

## Claims

1. An electroporation catheter comprising:
a shaft comprising:
an outer shaft element; and
an inner shaft element extending through the outer shaft element;
a plurality of splines forming a basket around a distal portion of the shaft, each spline extending between a proximal end that is coupled to the shaft and a distal end that is coupled to the shaft, , wherein the distal end of each spline is operatively connected to a distal end of the inner shaft element,
wherein the proximal end of each spline is connected to a distal end of the outer shaft element, and wherein the inner shaft element is slidable relative to the outer shaft element to adjust an effective diameter of the basket formed by the plurality of splines, wherein each spline of the plurality of splines comprises at least one energizable electrode; and
a balloon positioned within the basket formed by the plurality of splines, the balloon selectively inflatable to facilitate securing a position of the plurality of splines;
wherein a channel is defined between the inner shaft element and the outer shaft element, the channel in fluid communication with an interior of the balloon to facilitate selectively inflating the balloon using a fluid.

2. The electroporation catheter in accordance with claim 1, wherein each spline comprises:
a body comprising an elastic conductive material; and
an insulating material covering portions of the body, wherein at least one exposed portion of the body corresponds to the at least one selectively energizable electrode.

3. The electroporation catheter in accordance with claim 1, wherein each spline comprises at least one spread member corresponding to the at least one electrode, the at least one spread member comprising:
a first end;
a second end;
a first branch extending from the first end to the second end; and
a second branch extending from the first end to the second end, the first and second branches spaced apart from one another.

4. The electroporation catheter in accordance with claim 1, wherein each spline comprises at least one tapered member corresponding to the at least one electrode, the at least one tapered member comprising:
a first end;
a second end; and
at least one tapered portion positioned between the first end and the second end.

5. The electroporation catheter in accordance with claim 4, wherein the at least one tapered portion comprises a first tapered portion and a second tapered portion, and wherein the at least one tapered member further comprises a middle portion extending between the first tapered portion and the second tapered portion, the middle portion having a constant width.

6. The electroporation catheter in accordance with any preceding claim, wherein sliding the inner shaft element distally relative to the shaft element decreases the effective diameter, and wherein sliding the inner shaft element proximally relative to the shaft element increases the effective diameter.

7. The electroporation catheter in accordance with any preceding claim, wherein the inner shaft element defines a central lumen, the central lumen configured to receive at least one of contrast agent, a mapping catheter, a guidewire, and a shape sensing fiber.

8. The electroporation catheter in accordance with any preceding claim, wherein alternating splines have electrodes with alternating polarities.

9. An electroporation system comprising:
a generator; and
an electroporation catheter according to any preceding claim and further comprising a handle, wherein the shaft extends distally from the handle.
